(19) Europäisches Patentamt
European Patent Office
Office européen des brevets

(11) **EP 3 329 981 A2**

(12) **EUROPEAN PATENT APPLICATION**

(43) Date of publication:
**06.06.2018 Bulletin 2018/23**

(21) Application number: **17204603.9**

(22) Date of filing: **30.11.2017**

(51) Int Cl.:
*B01D 53/84* (2006.01)   *C12R 1/38* (2006.01)
*C12N 9/14* (2006.01)   *C12N 1/20* (2006.01)
*C12N 15/63* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.12.2016   KR 20160163892**

(71) Applicant: **Samsung Electronics Co., Ltd.**
**Gyeonggi-do 16677 (KR)**

(72) Inventors:
• **BYUN, Jongwon**
  **Suwon-si 16678 (KR)**

• **SONG, Seunghoon**
  **Suwon-si 16678 (KR)**
• **PARK, Jinhwan**
  **Suwon-si 16678 (KR)**
• **YANG, Dongsik**
  **Suwon-si 16678 (KR)**
• **LEE, Jinsuk**
  **Suwon-si 16678 (KR)**

(74) Representative: **Grünecker Patent- und Rechtsanwälte**
**PartG mbB**
**Leopoldstraße 4**
**80802 München (DE)**

(54) **EXHAUST GAS DECOMPOSITION SYSTEM AND EXHAUST GAS DECOMPOSITION COMPLEX SYSTEM INCLUDING THE SAME**

(57)    An exhaust gas decomposition apparatus or system that includes a bioreactor fluorine-containing compound is decomposed by contact with the first and second fluids in the bioreactor. The first fluid is supplied through a bioreactor inlet and is exhausted through the bioreactor outlet, and moves in a first direction in the bioreactor. The first or second fluid includes a biological catalyst such as an enzyme or recombinant microbe, while the other fluid includes a fluorine-containing compound. As a result, the fluorine-compounds is efficiently biologically remediated by the biological catalyst.

FIG. 1

<110>      Samsung Electronics Co., Ltd.

<120>      Exhaust gas decomposition system and exhaust gas decomposition
           complex system including the same

<130>      EP115104FZTRpau

<140>      not yet assigned
<141>      herewith

<150>      KR10-2016-0163892
<151>      2016-12-02

<160>      4

<170>      KopatentIn 2.0

<210>      1
<211>      236
<212>      PRT
<213>      Bacillus cereus

<400>      1
Met Lys Tyr Lys Val Ile Leu Phe Asp Val Asp Asp Thr Leu Leu Asp
 1               5                  10                  15

Phe Pro Glu Thr Glu Arg His Ala Leu His Asn Ala Phe Val Gln Phe
            20                  25                  30

Asp Met Pro Thr Gly Tyr Asn Asp Tyr Leu Ala Ser Tyr Lys Glu Ile
            35                  40                  45

Ser Asn Gly Leu Trp Arg Asp Leu Glu Asn Lys Met Ile Thr Leu Ser
        50                  55                  60

Glu Leu Ala Val Asp Arg Phe Arg Gln Leu Phe Ala Leu His Asn Ile
 65                  70                  75                  80

Asp Val Asp Ala Gln Gln Phe Ser Asp Val Tyr Leu Glu Asn Leu Gly
                85                  90                  95

Lys Glu Val His Leu Ile Glu Gly Ala Val Gln Leu Cys Glu Asn Leu
            100                 105                 110

Gln Asp Cys Lys Leu Gly Ile Ile Thr Asn Gly Tyr Thr Lys Val Gln
            115                 120                 125

Gln Ser Arg Ile Gly Asn Ser Pro Leu Cys Asn Phe Phe Asp His Ile
        130                 135                 140

Ile Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
            165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
            195                 200                 205

Asp Val Asn Pro Thr Tyr Glu Val Glu Ser Leu Leu Gln Ile Leu Glu

                210                     215                     220

        Ile Val Glu Val Ala Glu Glu Lys Val Ala Ser Phe
        225                     230                     235


        <210>    2
        <211>    711
        <212>    DNA
        <213>    Bacillus cereus

        <400>    2
        atgaaataca aagttatatt attcgacgta gatgatacat tattagattt ccctgaaacg        60

        gaaagacacg cattacataa tgcgtttgta cagtttgata tgcctacagg gtataatgat        120

        tatcttgcaa gctataaaga gattagtaat ggattatgga gagatttaga aaataaaatg        180

        attacgctaa gtgaattagc agtagatcga tttagacaat tatttgcact tcataatata        240

        gacgtagatg cacagcaatt tagtgatgta taccttgaaa atttagggaa ggaagtacat        300

        cttatagaag gcgcagtaca attatgtgaa aatctacaag attgcaagtt aggtattatt        360

        acgaatggat atacgaaggt gcaacaatca agaatcggaa attcaccttt atgtaatttc        420

        tttgatcaca ttattatttc tgaagaagtt ggtcatcaaa aaccagcacg tgagattttt        480

        gattatgcgt ttgagaagtt tgggattact gataaatcaa gcgtactaat ggttggagat        540

        tcgttaactt ctgatatgaa aggcggagaa gattacggca ttgatacgtg ttggtataat        600

        ccgagtttga aagaaaacgg gacagatgtt aacccgactt atgaagtgga gagtctgctc        660

        caaattttag aaattgtaga agtggcggaa gaaaaggtag cttcatttta a        711


        <210>    3
        <211>    40
        <212>    DNA
        <213>    Artificial Sequence

        <220>
        <223>    primer


        <400>    3
        aagaaggaga tataccatga aatacaaagt tatattattc        40


        <210>    4
        <211>    41
        <212>    DNA
        <213>    Artificial Sequence

        <220>
        <223>    primer


        <400>    4
        gcattatgcg gccgcaagct ttaaaatgaa gctacctttt c        41

**Description**

BACKGROUND

1. Field

The present disclosure relates to an exhaust gas decomposition system and an exhaust gas decomposition complex system including the same. In addition, the present disclosure relates to a strain KCTC 13107BP of *Pseudomonas saitens* (hereinafter, also referred to as "SF1 strain"), which is capable of reducing a concentration of hydrofluorocarbon or fluorocarbon in a sample, and a method of reducing a concentration of hydrofluorocarbon or fluorocarbon in a sample by using the strain.

2. Description of the Related Art

Greenhouse gases, such as fluorinated gases, exhausted from industrial processes including semiconductor processes, cause global warming or other environmental problems, and thus a remedial process, for example, a decomposition treatment, is required. Often, exhaust gas is treated by a high-temperature or catalytic chemical decomposition method. For example, a method of decomposing exhaust gas at a high temperature of at least 1,400 °C, or a catalytic thermal oxidation method of decomposing exhaust gas by oxidization of exhaust gas by using a metallic catalyst, e.g., $Ce/Al_2O_3$, is often used. Such a chemical decomposition method requires large-capacity facilities and involves massive energy consumption.

Therefore, there is a need for new environmentally friendly and economical decomposition methods for decomposing exhaust gas.

In this regard, there has been a demand for a microorganism capable of reducing a concentration of fluorinated methane in a sample.

SUMMARY

Provided is an exhaust gas decomposition system for improving a decomposition rate of a fluorine-containing compound.

Provided is an exhaust gas decomposition complex system including the exhaust gas decomposition system.

Provided is a microorganism of the genus *Pseudomonas,* the microorganism being capable of reducing a concentration of fluorinated methane in a sample.

Provided is a method of removing fluorinated methane in a sample by using the microorganism of the genus *Pseudomonas* that is capable of reducing a concentration of fluorinated methane in a sample.

Additional aspects will be set forth in part in the description which follows and, in part, will be apparent from the description.

According to an aspect of an embodiment, there is provided an exhaust gas decomposition system and system including:

one or more reactors, each including one or more first inlets and one or more first outlets,
wherein a fluorine-containing compound is decomposed by contact between a first fluid and a second fluid in each of the one or more reactors,wherein the first fluid is supplied through the one or more first inlets and is exhausted through the one or more outlets,
and flows in a first direction in each of the one or more reactors, and wherein one of the first fluid and the second fluid includes a biological catalyst, and the other includes a fluorine-containing compound.

According to an aspect of another embodiment, there is provided an exhaust gas decomposition system and system e.g., designed as an exhaust gas decomposition complex including: the exhaust gas decomposition system; a first supplier for supplying the first fluid into the exhaust gas decomposition system; a second supplier for supplying the second fluid into the exhaust gas decomposition system; and a collecting device for collecting a decomposition product discharged from the exhaust gas decomposition system.

According to an aspect of another embodiment, there is provided a KCTC 13107BP strain of *Pseudomonas saitens,* the strain being capable of reducing a concentration of tetrafluoromethane ($CF_4$) in a sample.

According to an aspect of another embodiment, there is provided a method of reducing a concentration of fluorinated methane in a sample, the method including:

reducing a concentration of fluorinated methane in a sample by contact between the KCTC 13107BP strain of *Pseudomonas saitens* and the sample, the sample including fluorinated methane represented by $CH_nF_{4-n}$ (where n is

an integer of 0 to 3).

BRIEF DESCRIPTION OF THE DRAWINGS

These and/or other aspects will become apparent and more readily appreciated from the following description of the embodiments, taken in conjunction with the accompanying drawings in which:

FIG. 1 is a schematic diagram showing an exhaust gas decomposition system illustrating flow of a first fluid in a first direction and optional recirculation according to an embodiment;
FIG. 2 is a schematic diagram showing an exhaust gas decomposition system illustrating flow of a second fluid in a second direction and optional recirculation according to another embodiment;
FIG. 3 is a schematic diagram showing an exhaust gas decomposition system of Fig. 1 having a fluid reaction zone in an upper portion and a fluid collection zone according to another embodiment;
FIG. 4 is a schematic diagram showing an exhaust gas decomposition system having a structure that increases the area of contact between a first and second fluids according to another embodiment;
FIG. 5 is a schematic diagram showing an exhaust gas decomposition system having multiple inlets and sprayers, according to another embodiment;
FIG. 6 is a schematic diagram showing an exhaust gas decomposition system having multiple sprayers connected by a line from a single inlet according to another embodiment;
FIG. 7 is a schematic diagram showing the geometry of an exhaust gas decomposition system according to another embodiment;
FIG. 8 is a schematic diagram showing an exhaust gas decomposition system according to another embodiment;
FIG. 9 is a schematic diagram showing an exhaust gas decomposition system including a plurality of reactors that are connected to one another in series according to another embodiment;
FIG. 10 is a schematic diagram showing an exhaust gas decomposition system including a plurality of reactors that are connected to one another in parallel according to another embodiment;
FIG. 11 is a schematic diagram showing an exhaust gas decomposition complex system with first and second suppliers and collectors, in accordance with an embodiment;
FIG. 12 is a schematic diagram showing a reactor used in Examples 1 and 4;
FIG .13 is a schematic diagram showing a reactor used in Examples 2 and 5;
FIG. 14 is a schematic diagram showing a reactor used in Examples 3 and 6;
FIG. 15 shows a vector map of a pET-BC HAD vector; and
FIG. 16 is a diagram showing the phylogenetic tree of a separated microorganism.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The term "increase in activity" or "increased activity", as used herein, refers to a detectable increase in an activity of 2-haloacid dehalogenase (HAD) in a cell. For instance, an "increase in activity" or "increased activity" may refer to an activity level of HAD in a modified (for example, genetically engineered) cell that is higher than that of a comparative cell of the same type that does not have a given genetic modification (e.g., original or "wild-type" cell). Activity of HAD in a modified or engineered cell may be increased by any amount, such as by about 5% or more, about 10% or more, about 15% or more, about 20% or more, about 30% or more, about 50% or more, about 60% or more, about 70% or more, or about 100% or more than an activity of a cell of the same type without a given genetic modification, e.g., activity of HAD in a non-engineered or "wild-type" cell. A cell having an increased activity of HAD may be identified by using any method known in the art.

An increase in an activity of HAD in a cell may be achieved by an increase in expression or specific activity. The increase in expression may be caused by introduction of an exogenous polynucleotide encoding the HAD enzyme into a cell, by otherwise increasing of the copy number of a gene encoding a HAD enzyme, or by modification of a regulatory region of the polynucleotide encoding the HAD enzyme so as to increase expression levels. A microorganism into which the polynucleotide encoding the enzyme is introduced may be a microorganism that may or may not already include the polynucleotide (e.g., gene). The polynucleotide encoding the enzyme may be operably linked to a regulatory sequence that enables expression thereof, for example, a promoter, a polyadenylation site, ribosomal binding site, start and stop codons, or a combination thereof. The exogenous polynucleotide may be homologous (i.e., native to the microorganism into which it is introduced) or heterologous (i.e., not natively present in the organism).

The term "increase of the copy number", as used herein, refers to a case in which the copy number is increased by introduction of another copy of an existing gene, amplification of an endogenous gene, or introduction of a gene that does not normally exist in the non-engineered cell. The introduction of the gene may be mediated by a vehicle, such as a vector. The introduction may be a transient introduction in which the gene is not integrated into a genome (e.g. via an unstable episome), or introduction that results in stable integration of the gene into the genome (e.g. episomal or chromosomal). The introduction may be performed by, for example, introducing a vector into the cell, the vector including a polynucleotide encoding a target polypeptide, and then, replicating the vector in the cell, or by integrating the polynucleotide into the genome.

The introduction of the gene may be performed by a known method, for example, transformation, transfection, or electroporation. The gene may be introduced via a vehicle or as it is. The term "vehicle" (or, alternatively "vector"), as used herein, refers to a nucleic acid molecule that is able to deliver other nucleic acids linked thereto into a cell. As a nucleic acid sequence mediating introduction of a specific gene, the vehicle used herein may be a nucleic acid construct, such as a vector or a cassette, a plasmid vector, a virus-derived vector, such as a replication-defective retrovirus, adenovirus, adeno-associated virus, or a combination thereof.

The term "parent cell" refers to an original cell, for example, a non-genetically engineered cell of the same type as an engineered microorganism. With respect to a particular genetic modification, the "parent cell" may be a cell that lacks the particular genetic modification, but is identical in all other respects. Thus, the parent cell may be a cell that is used as a starting material to produce a genetically engineered microorganism having an increased activity of a given protein (e.g., a protein having an amino acid sequence identity of about 90% or higher with respect to 2-haloacid dehalogenase (HAD)). The same comparison is also applied to other genetic modifications.

The term "gene", as used herein, refers to a nucleic acid fragment encoding a particular protein, and may or may not include a regulatory sequence, e.g., a-non coding sequence 5' and/or a 3' of the coding sequence.

The term "sequence identity" of a polynucleotide or a polypeptide, as used herein, refers to a degree of identity between bases or amino acid residues of sequences obtained after the sequences are aligned so as to best match in certain comparable regions. The sequence identity is a value that is measured by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear so that the number of matching locations may be obtained, dividing the number of matching locations by the total number of locations in the comparable regions (that is, the size of a range), and multiplying a result of the division by 100 to obtain the percentage of the sequence identity. The percentage of the sequence identity may be determined using a known sequence alignment or comparison program, for example, BLASTN (NCBI), BLASTP (NCBI), CLC Main Workbench (CLC bio), MegAlign™ (DNASTAR Inc), etc.

Various levels of sequence identity may be used to identify various types of polypeptides or polynucleotides having the same or similar functions or activities. For example, the sequence identity may include a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100%.

The term "genetic modification", as used herein, refers to an artificial alteration in a constitution or structure of the genetic material of a cell.

Hereinafter, embodiments of an exhaust gas decomposition system, an exhaust gas decomposition complex system including the same, and methods of using the system or system will be described in detail.

The term "exhaust gas" used herein refers to all kinds of gases including a fluorine-containing compound exhausted from fixed or mobile machinery, equipment, or the like. The exhaust gas may be a mixture containing liquid or solid particles in addition to pure gas.

According to an embodiment, an exhaust gas decomposition system may include: one or more bioreactors, each of which includes one or more first inlets (inlets for introduction of a first fluid) and one or more first outlets (outlets for expelling a first fluid). Each bioreactor can further comprise one or more second inlets (inlets for introduction of a second fluid) and one or more second outlets (outlets for expelling a second fluid). Thus, each bioreactor of the apparatus or system can include a plurality of inlets (e.g., at least 1, 2, 3, 4, or more inlets) and a plurality of outlets (e.g., at least 1, 2, 3, 4, or more outlets. In each of the one or more bioreactors, a fluorine-containing compound may be decomposed by contact between a first fluid and a second fluid. The one or more bioreactors may comprise a first flow path for the first fluid, said first flow path running from at least one of the one or more first inlets in a first direction through the bioreactor (and the second fluid in the bioreactor) to at least one of the one or more first outlets. The one or more bioreactors may comprise a second flow path for the second fluid, said second flow path running from at least one of the one or more second inlets in a second direction through the bioreactor (and the second fluid in the bioreactor) to at least one of the one or more second outlets. The first fluid may be supplied through the one or more first inlets (e.g. from a supply via a line) and exhausted through the one or more first outlets, and may flow in a first direction, and the second fluid may be supplied through the one or more second inlets (e.g. from a supply via a line) and exhausted through the one or more second outlets, and may flow in a second direction generally opposite the first direction. One of the first fluid and the second fluid may include a biological catalyst that catalyzes decomposition of the fluorine-containing compound, and the other may include a fluorine-containing compound. The bioreactor can provide a bed on which a thin film of the first fluid formed and it flows in a first direction. For example, the bed can be inner wall of the reactor. In one embodiment, the first fluid may form a fluid thin film in a fluid reaction zone disposed at an upper portion of an inner space of at least one of the one or more bioreactors, and the fluid thin film of the first fluid may be in contact with the second fluid. In another embodiment, the fluid thin film may be disposed on an inner wall of at least one of the one or more bioreactors.

Since the fluorine-containing compound is decomposed by using a biological catalyst in the exhaust gas decomposition system, the fluorine-containing compound may be decomposed in an environmentally friendly manner without involvement of a high temperature and heat, and without using excessive energy. In addition, due to a continuous and repeated flowing of at least one of the first fluid and the second fluid in each of the one or more reactors in the exhaust gas decomposition system by circulation or the like, the contact time between the first fluid and, in certain embodiments, the second fluid may be increased, and accordingly, a decomposition rate of the fluorine-containing compound may be improved.

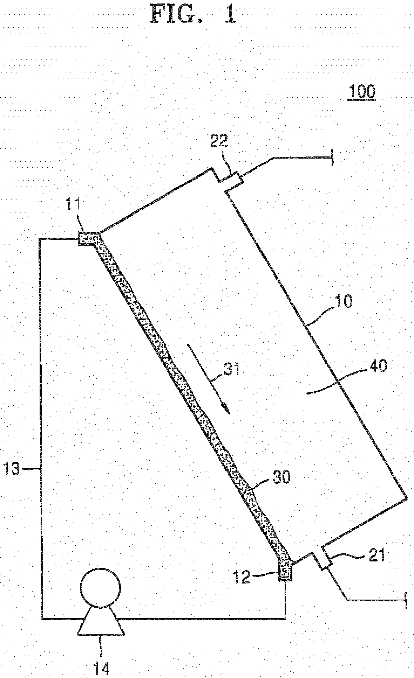

Referring to FIG. 1, an exhaust gas decomposition system 100 includes one or more reactors 10, each of which includes one or more first inlets 11, one or more first outlets 12. A first fluid comprising a biological catalyst 30 may be supplied through the one or more first inlets 11 and exhausted through the one or more first outlets 12, and may flow in a first direction 31 within each of the one or more reactors 10. In each of the one or more reactors 10, a second fluid may reside or flow that contains a fluorine-containing compound, and the fluorine-containing compound in the second fluid may be decomposed by contact between first fluid 30 and second fluid 40. In some embodiments, the second fluid (not shown in Fig. 1) may be supplied through the one or more second inlets 21 and exhausted through the one or more second outlets 22, and may flow in a second direction 41 within each of the one or more reactors 10. Alternatively, the first fluid 30 can comprise the fluorine-containing compound, and the second fluid 40 may include a biological catalyst. The number of the first inlets 11 and the first outlets 12 are not particularly limited, but depending on the required reaction conditions, the respective numbers thereof may be one or more.

Referring to FIGS. 1 to 7, in the exhaust gas decomposition system 100, the first fluid 30 may be liquid containing a biological catalyst, and the second fluid 40 may be a gas including a fluorine-containing compound.

Referring to FIGS. 1 to 6, each of the one or more reactors 10 in the exhaust gas decomposition system 100 may further include a first circulation line 13 for re-supplying at least a portion of the first fluid 30 discharged from the one or more first outlets 12 back to the one or more first inlets 11. The first circulation line 13 may be operated by, for example, a first circulation pump 14, but embodiments are not limited thereto. Any device capable of circulating fluid, such as fan, may be used. Due to the recirculation of at least a portion of the first fluid 30 back to each of the one or more reactors 10 in the exhaust gas decomposition system 100, the first fluid 30 and the second fluid 40 may be in continuous contact with each other, thereby increasing the contact time and decomposition rate of the fluorine-containing compound. For example, referring to FIG. 1, in the exhaust gas decomposition system 100, the first fluid 30 may circulate through each of the one or more reactors 10 and along the first circulation line 13, whereas the second fluid 40 may be present only inside each of the one or more reactors 10. The second fluid 40 may be supplied to each of the one or more reactors 10 through a second inlet 21, and after the completion of the reaction, the second fluid 40 may be discharged from each of the one or more reactors 10 through a second outlet 22.

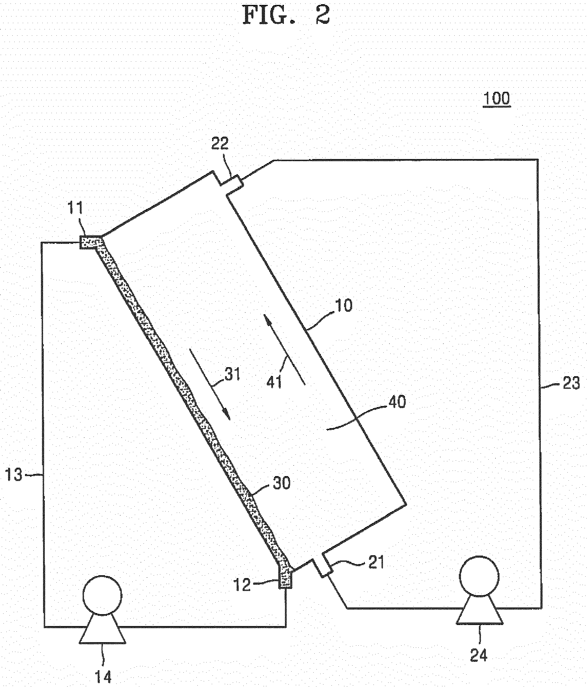

Referring to FIG. 2, each of the one or more reactors 10 in the exhaust gas decomposition system 100 may further include a second inlet 21 and a second outlet 22. The second fluid 40 may be supplied to each of the one or more reactors 10 through the second inlet 21, discharged to the outside of each of the one or more reactors 10 through the second outlet 22, and may flow in a second direction 41 within each of the one or more reactors 10. For example, the second direction 41 in which the second fluid 40 flows may be different from the first direction 31 in which the first fluid 30 flows. For example, the second direction 41 in which the second fluid 40 flows may be a direction opposite to the first direction 31 in which the first fluid 30 flows. In each of the one or more reactors 10 in the exhaust gas decomposition system 100, due to the flow of the first fluid 30 and the second fluid 40 in different or in opposite directions, a substantial area of contact between the first fluid 30 and the second fluid 40 may increase, thereby improving the decomposition rate of the fluorine-containing compound.

Each of the one or more reactors 10 in the exhaust gas decomposition system 100 may further include a second circulation line 23 for re-supplying at least a portion of the second fluid 40 discharged from the second outlet 22 back to the second inlet 21. The fluid in the second circulation line 23 may impelled by, for example, a second circulation pump 24, but embodiments are not limited thereto. Any device capable of circulating fluid in the art, such as fan, may be used. Due to the circulation of the first fluid 30 through each of the one or more reactors 10 and along the first circulation line 13 and the second fluid 40 through each of the one or more reactors 10 and along the second circulation line 23, contact time between the first fluid 30 and the second fluid 40 may be increased, and accordingly, the decomposition rate of the fluorine-containing compound may be improved.

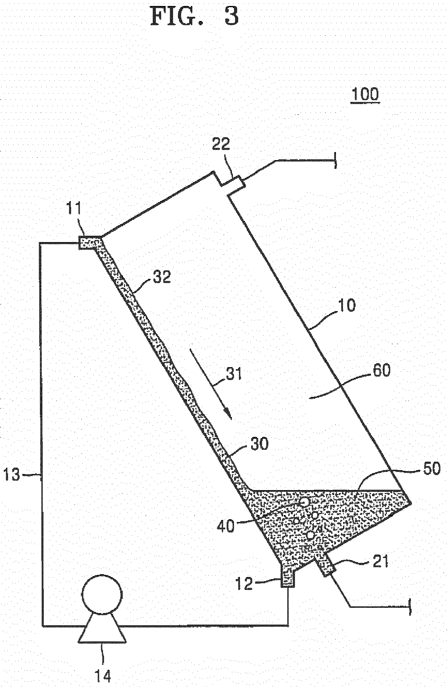

Referring to FIG. 3, a fluid collection zone 50 is disposed at a lower (e.g., in the bottom half as measured along an axis of an elongate reactor) portion of the interior of one or more reactors 10 and a fluid reaction zone 60 is disposed at an upper (e.g., in the top half as measured along an axis of an elongate reactor) portion of the interior of each of the one or more reactors 10 in the exhaust gas decomposition system 100, so the first fluid 30 and the second fluid 40 may contact each other, thereby decomposing the fluorine-containing compound. In the inside, i.e., inner space or lumen, of each of the one or more reactors 10, the fluid collection zone 50 may be disposed at a bottom (e.g., in the bottom half as measured along the axis) portion, and the fluid reaction zone 60 may be disposed above the fluid collection zone 50. The upper portion of each of the one or more bioreactors 10 correspond to a region near inlet 11 and lower portion of each of the one or more bioreactors 10 correspond to a region near outlet 12. The first fluid 30 supplied to each of the one or more reactors 10 through the one or more first inlets 11 may be collected in the fluid collection zone 50. Here, a size and a shape of the fluid collection zone 50 are not particularly limited, but may be determined by a total volume and a shape of the collected first fluid 30. The fluid reaction zone 60 may occupy the inner space of each of the one or more reactors 10, except for the fluid collection zone 50. At least a portion of the first fluid 30 collected in the fluid collection zone 50 may be discharged to the outside of each of the one or more reactors 10 through the first outlet 12. Since the fluid collection zone 50 includes the second inlet 21, the second fluid 40 supplied into each of the one or more reactors 10 may contact the first fluid 30 while the second fluid 40 passes, in the form of bubbles, through the collected the first fluid 30, thereby decomposing the fluorine-containing compound. In addition, in the fluid reaction zone 60, the second fluid 40 passing through the fluid collection zone 50 may contact the first fluid 30 supplied into each of the one or more reactors 10 through the first inlet 11, thereby decomposing the fluorine-containing compound.

Referring to FIG. 3, in the fluid reaction zone 60 disposed at an upper portion of the interior of the exhaust gas decomposition system 100, a fluid thin film 32 containing the first fluid 30 may contact the second fluid 40. A fluid thin film as used herein means a layer of fluid on a surface having a width or length dimension parallel to the surface that is substantially greater (e.g., 10x or more, 100x or more, or 1000x or more) than its depth perpendicular to the surface. By way of illustration, and without limitation, a fluid thin film on a surface might have a depth of a few micrometers (e.g., 5 um or 10 um) to tens or hundreds of micrometers (e.g., 50 um, 100 um, 500 um, 1,000 um or 5000 um). The fluid thin film may has a thickness of 0.1mm to 10mm, or 0.5 or 5mm. When the first fluid 30 in the form of the fluid thin film 32 contacts the second fluid 40, the area of contact between the first fluid 30 and the second fluid 40 may increase because the thin film of first fluid has a greater surface area to contact the second fluid. In this regard, since an amount of the first fluid 30 contacting the second fluid 40 increases, the residence time of the first fluid 30 contacting the second fluid 40 in each of the one or more reactors 10 may also increase, thereby improving the decomposition rate of the fluorine-containing compound. The fluid thin film 32 may be disposed on the inner wall of each of the one or more reactors 10 such that the fluid thin film 32 may flow while covering the inner wall. As the inner wall of each of the one or more reactors 10 is coated by the fluid thin film 32, the residence time of the first fluid 30 in each of the one or more reactors 10 may also increase.

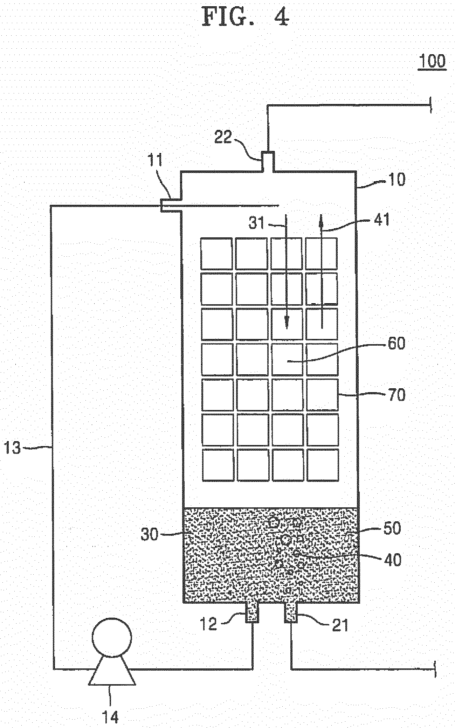

Referring to FIG. 4, each of the one or more reactors 10 of the exhaust gas decomposition system 100 may further include a structure 70 that increases the area of contact between the first fluid 30 and the second fluid 40. For example, the structure 70 may be at least one of a filling material and a reflux pipe, but embodiments are not limited thereto. The filling material may be a particulate material such as inorganic porous beads or organic porous beads. The filling material may be filler and it may be a bed for a formation of thin film of the first fluid. Any structure in the art capable of increasing the area of contact between the first fluid 30 and the second fluid 40 may be used. When the structure 70 is a filling material, any open structure may be used wherein the open structure is a regularly or irregularly structured hollow structure having high porosity due to having formed therein many interstices and large passages for the flow of fluids. The large passage means a passage in which a diameter of the passage is larger than a thickness of the thin film of the first fluid 30 that flows through the passage. The structure 70 may be connected and fixed to each of the one or more reactors 10. Alternatively, the structure 70 may be simply put into the lumen of the one or more reactors 10, recovered after a period of time, and then separated from the one or more reactors 10. For example, the structure 70 may be porous. For example, the structure 70 may include porous polymeric particles, such as porous polypropylene particles, porous inorganic particles, such as zeolite, and the like, but embodiments are not limited thereto. Any porous material available in the art may be used as the structure 70. The size of the porous polymeric particles and the porous inorganic particles is not particularly limited. For example, the porous polymeric particles and the porous inorganic particles may have a particle size in a range of about $1 \text{ mm}^3$ to about $1,000 \text{ cm}^3$, about $1 \text{ mm}^3$ to about $100 \text{ cm}^3$, about $1 \text{ mm}^3$ to about $10 \text{ cm}^3$, about $1 \text{ mm}^3$ to about $1 \text{ cm}^3$, or about $1 \text{ mm}^3$ to about $0.1 \text{ cm}^3$. The porosity of the porous polymeric particles and the porous inorganic particles is not particularly limited. For example, the porous polymeric particles and the porous inorganic particles may have porosity in a range of about 1% to about 99%, about 5% to about 95%, about 10% to about 90%, about 20% to about 80%, or about 30% to about 70%. The porosity refers to the volume occupied by pores in the total volume of the particles.

The volume occupied by the construct 70 within the entire volume of the reactor 10 is not particularly limited, but for example, the construct 70 may account for about 1% to about 99%, about 5% to about 95%, about 10% to about 90%, about 20% to about 80%, or about 30% to about 70% of the entire volume of the reactor 10. When the area of contact between the first fluid 30 and the second fluid 40 increases by disposing the structure 70 within the reactor 10, the decomposition rate of the fluorine-containing compound may be improved.

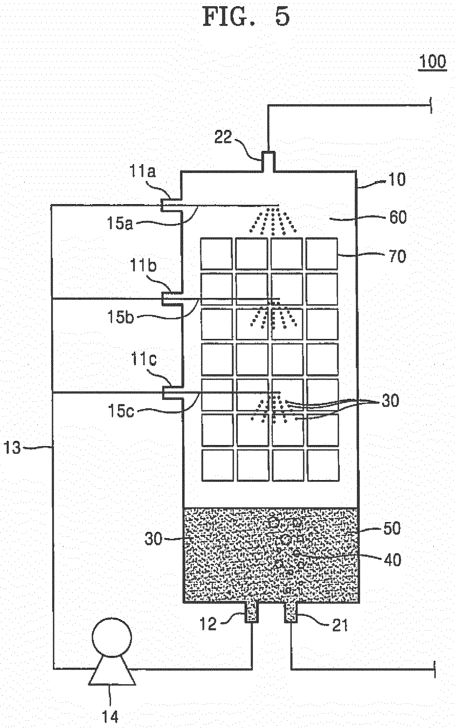

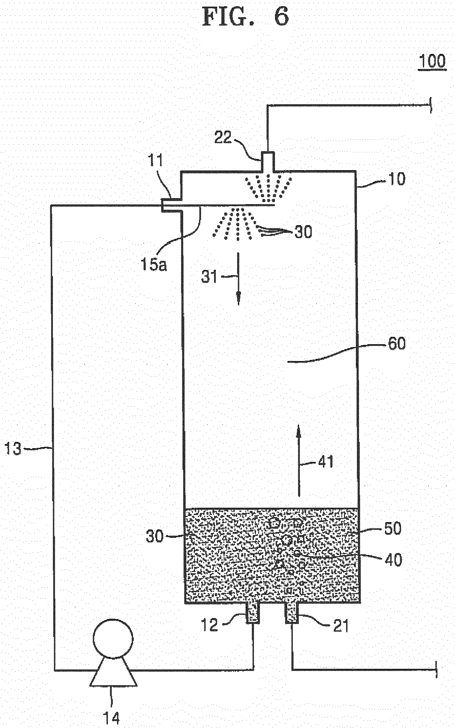

Referring to FIGS. 5 and 6, one or more first inlets 11 a, 11 b, and 11c are connected to the fluid reaction zone 60 at an upper portion of the interior of the reactor 10 in the exhaust gas decomposition system 100, thereby supplying the first fluid 30 to the reactor 10 through the one or more first inlets 11a, 11b, and 11c. As the first fluid 30 is supplied to the reactor 10 through the one or more first inlets 11a, 11b, and 11c, the first fluid 30 may be uniformly supplied to the fluid reaction zone 60 at an upper portion of the interior of the reactor 10. In addition, referring to FIG. 5, a fluid thin film (not shown) having a uniform thickness may be formed on a surface of the fluid reaction zone 60 at an upper portion of the interior of the reactor 10, thereby obtaining a uniform decomposition rate of the fluorine-containing gas. In addition, the reactor 10 may further include one or more sprayers 15a, 15b, and 15c that are connected to the one or more first inlets 11a, 11b, and 11c, respectively, for spraying the first fluid 30 into the fluid reaction zone 60. Here, the direction in which the sprayers 15a, 15b, and 15c each spray the first fluid 30 is not limited, and may each spray the first fluid 30 in top, bottom, left, and right directions while rotating.

Referring to FIGS. 3 to 6, in the exhaust gas decomposition system 10, the first fluid 30 may be collected in the fluid collection zone 50 at a bottom portion of the interior of each of the one or more reactors 10, and the second fluid 40 supplied into the reactor 10 through the second inlet 21 may pass, in the form of bubbles, through the collected first fluid 30. The second fluid 40 may flow to the fluid reaction zone 60 disposed at a top portion of the interior of each of the one or more reactors 10, and then, may be discharged out of each of the one or more reactors 10 through the second outlet 22. The second fluid 40 may have a wide area of contact with the first fluid 30 in the fluid reaction zone 60, and thus, the fluorine-containing compound may be decomposed mainly in the fluid reaction zone 60.

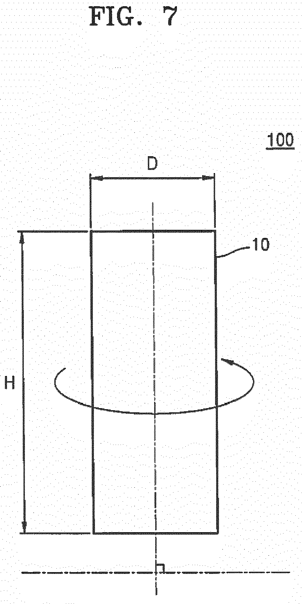

Referring to FIG. 7, in the exhaust gas decomposition system 100, an aspect ratio of the reactor 10, i.e., a ratio of a diameter D to a height H of each of the one or more reactors 10 may be 2 or more. When the aspect ratio of each of the one or more reactors 10 is 2 or more, the residence time of the first fluid 30 and the second fluid 40 in the reactor 10 may increase, thereby improving the decomposition rate of the fluorine-containing compound. For example, the aspect ratio of the reactor 10 may be 5 or more. For example, the aspect ratio of the reactor 10 may be 10 or more. For example, the aspect ratio of the reactor 10 may be 15 or more. For example, the aspect ratio of the reactor 10 may be 20 or more. For example, the aspect ratio of the reactor 10 may be 50 or less.

Referring to FIG. 7, in the exhaust gas decomposition system 100, the reactor 10, e.g., a longitudinal axis H of the reactor 10, may be arranged at an angle of about 30° to about 150° (e.g., about 30° to less than 90°, or greater than 90° to about 150°) with respect to the surface of the earth. For example, the reactor 10 may be arranged at an angle of about 50° to about 130° (e.g., about 50° to less than 90°, or greater than 90° to about 130°) with respect to the surface of the earth. For example, the reactor 10 may be arranged at an angle of about 70° to about 110° (e.g., about 70° to less than 90°, or greater than 90° to about 110°) with respect to the surface of the earth. For example, the reactor 10 may be arranged at an angle of about 80° to about 100° (e.g., about 80° to less than 90°, or greater than 90° to about 100°) with respect to the surface of the earth. For example, the reactor 10 may be arranged at an angle of about 50° to about 90° or less than 90° with respect to the surface of the earth. In the exhaust gas decomposition system 100, the reactor 10, e.g., a longitudinal axis H of the reactor 10, may be arranged at an angle of about 90°. Further, when exhaust gas decomposition system 100, the reactor 10, e.g., a longitudinal axis H of the reactor 10, may be arranged at an angle of about 90°, the inner wall of the reactor 10 may have may be arranged at an angle of less than 90° to form a bed within the reactor 10. Within the range of the angle at which the reactor 10 is disposed, a fluid thin film (not shown) containing the first fluid 30 may be well formed on an inner wall of the reactor 10 over a large area, thereby improving the decomposition rate of the fluorine-containing compound.

Referring to FIG. 7, in the exhaust gas decomposition system 100, the reactor 10 may rotate. The system may comprise rotating member, e.g. a hub, axle, bearing or flange, and/or a rotation actuator, such as a motor or gear. For example, in the exhaust gas decomposition system 100, the reactor 10 may rotate on a longitudinal axis H of the reactor 10. Here, a direction and a speed of rotation of each of the one or more reactors 10 may be appropriately selected within a range in which the area of contact between the first fluid 30 and the second fluid 40 in each of the one or more reactors 10 may increase. For example, the speed at which the reactor 10 rotates may be in a range of about 0.01 revolutions per minute (rpm) to about 100 rpm. For example, the speed at which each of the one or more reactors 10 rotates may be in a range of about 0.1 rpm to about 10 rpm.

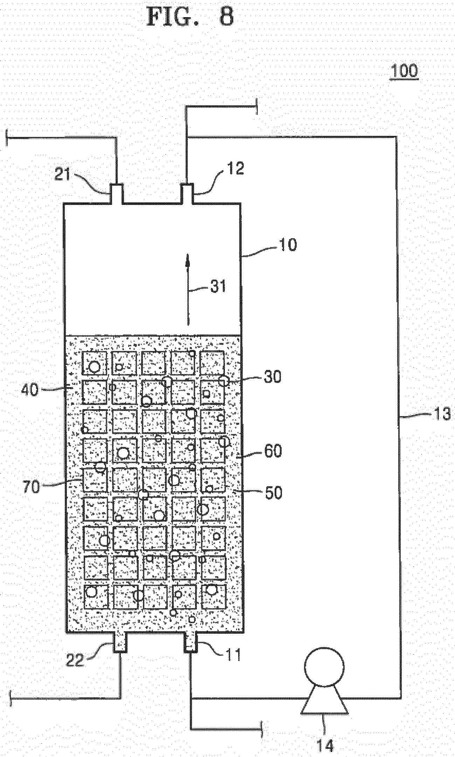

Referring to FIG. 8, in the exhaust gas decomposition system 100, the first fluid 30 may be gas including a fluorine-containing compound, and the second fluid 40 may be liquid containing a biological catalyst.

In the exhaust gas decomposition system 100 of FIG. 8, the first fluid 30 may be gas and the second fluid 40 may be liquid, wherein the second fluid does not recirculate. In embodiments of the system of Figures 1-7, liquid which is the first fluid 30 circulates in the exhaust gas decomposition system. In the system of FIG. 8, the liquid which is the second fluid 40 does not circulate during a reaction.

In the exhaust gas decomposition system 100 of FIG. 8, the second fluid 40 may be in the fluid collection zone 50 disposed at a bottom portion of the interior of the reactor 10, and the first fluid 30 supplied into the reactor 10 through the first inlet 11 may pass, in the form of bubbles, through the second fluid 40. The second fluid 40 may flow to an upper portion of the interior of each of the one or more reactors 10, and then, may be discharged from each of the one or more reactors 10 through the second outlet 22. As the first fluid 30 passes, in the form of bubbles, through the second fluid 40, the fluorine-containing compound may be decomposed by the contact between the first fluid 30 and the second fluid 40. That is, in the reactor 10 of the exhaust gas decomposition system 100 of FIG. 8, the fluid collection zone 50 is the same as the fluid reaction zone 60 in its functions. In addition, in the exhaust gas decomposition system 100 of FIG. 8, the reactor 10 may further include the first circulation line 13 for re-supplying at least a portion of the first fluid 30 discharged from the first outlet 12 back through the first inlet 11. In the exhaust gas decomposition system 100 of FIG. 8, due to the circulation of at least a portion of the first fluid 30 back to reactor 10, the first fluid 30 and the second fluid 40 may be in continuous contact with each other, thereby increasing the contact time thereof, and in this regard, the decomposition rate of the fluorine-containing compound may be improved. For example, in the exhaust gas decomposition system 100 of FIG. 8, the first fluid 30 may circulate through the reactor 10 and along the first circulation line 13, whereas the second fluid 40 may be present only inside the reactor 10.

Referring to FIG. 8, at a bottom portion of the interior of the reactor 10 in the exhaust gas decomposition system 100, the structure 70 that increases the contact area between the first fluid 30 and the second fluid 40 may be further included. The structure 70 is the same as described above. The additional inclusion of the structure 70 increases the area of contact between the first fluid 30 and the second fluid 40 at a bottom portion of the interior of the reactor 10 and may lead to improvement of the decomposition rate of the fluorine-containing compound.

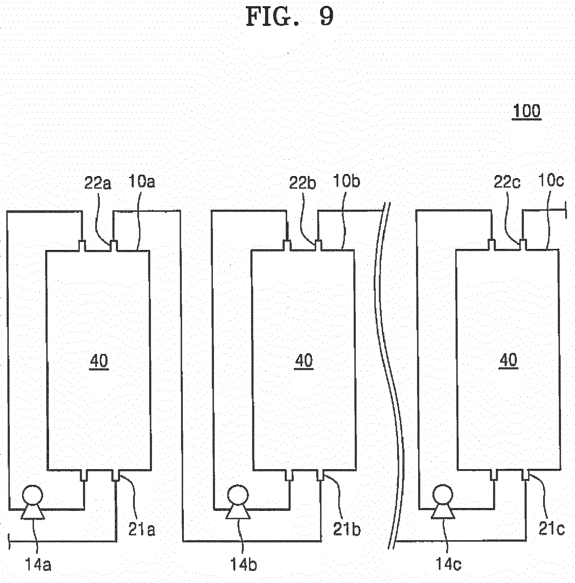

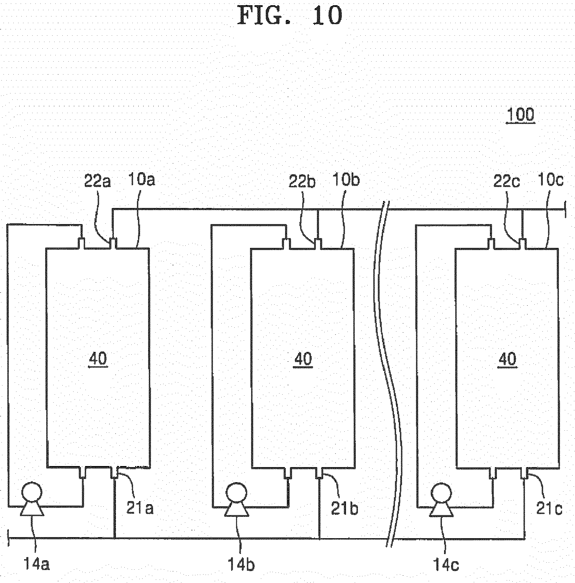

Referring to FIGS. 9 and 10, in the exhaust gas decomposition system 100, a plurality of reactors 10a, 10b, and 10c may be connected to one another in series or in parallel, thereby improving the decomposition rate of the fluorine-containing compound.

Referring to FIG. 9, in the plurality of reactors 10a, 10b, and 10c that are connected one another in series in the exhaust gas decomposition system 100, the first fluid (not shown) or the second fluid 40 discharged from one reactor (e.g., reactor 10a) may be sequentially supplied to another reactor (e.g., reactor 10b). As the number of the reactors connected to one another in series increases, the time and/or area of contact between the first fluid (not shown) and the second fluid 40 may also increase, thereby improving the decomposition rate of the fluorine-containing compound. For example, the second fluid 40 may be supplied to one reactor (e.g., reactor 10a) through one second inlet (e.g., second inlet 21a) and discharged through one second outlet (e.g., second outlet 22a), and then, may be supplied again to another reactor (e.g., reactor 10b) through another second inlet (e.g., second inlet 21b) and discharged again out from the reactor 10b through another second outlet (e.g, second outlet 22a). After passing through the plurality of reactors 10a, 10b, and 10c in this manner, the second fluid 40 may be supplied to a final reactor (e.g., reactor 10c) through a second inlet (e.g., second inlet 21c) and discharged through a second outlet (e.g., second outlet 22c).

Referring to FIG. 10, in a plurality of reactors 10a, 10b, and 10c that are connected to one another in parallel in the exhaust gas decomposition system 100, the first fluid (not shown) or the second fluid may be simultaneously supplied to each of the plurality of reactors 10a, 10b, and 10c, and then, may be simultaneously discharged out from each of the plurality of reactors 10a, 10b, and 10c. As the number of reactors that are connected to one another in parallel increases, the time and/or area of contact between the first fluid (not shown) and the second fluid 40 may also increase, thereby improving the decomposition rate of the fluorine-containing compound. For example, the second fluid 40 may be simultaneously supplied to the plurality of reactors 10a, 10b, 10c through a plurality of second inlets 21a, 21b, and 21c, respectively, and may be simultaneously exhausted through a plurality of second outlets 22a, 22b, and 22c.

Referring to FIGS. 1 to 10, an inside temperature of the reactor 10 of the exhaust gas decomposition system 100 may be about 50°C or less. Since a biological catalyst is used in the reactor 10, the fluorine-containing compound may be decomposed at low temperatures of 50°C or less. For example, the inside temperature the reactor 10 of the exhaust gas decomposition system 100 may be about 45°C or less. For example, the inside temperature of the reactor 10 of the exhaust gas decomposition system 100 may be about 40°C or less, For example, the inside temperature of the reactor 10 of the exhaust gas decomposition system 100 may be about 35°C or less. For example, the inside temperature of the reactor 10 of the exhaust gas decomposition system 100 may be about 30°C or less. For example, the inside temperature of the reactor 10 of the exhaust gas decomposition system 100 may be about may be in a range of about 20°C to about 50°C. When the inside temperature of the reactor 10 is within the above ranges, the decomposition rate of the fluorine-containing compound may be improved. Temperature control can be achieved via a suitable thermal control device (e.g., a thermostat and thermocouple with a microcontroller).

Referring to FIGS. 1 to 10, the decomposition rate of the fluorine-containing compound after about 70 hours in the exhaust gas decomposition system 100 may be about 30% or more. That is, the amount of the fluorine-containing compound included in the reactor 10 of the exhaust gas decomposition system 100 may be reduced to about 70% or less relative to the initial amount of the fluorine-containing compound, after about 70 hours from the initial introduction time of the fluorine-containing compound into the reactor. For example, the decomposition rate of the fluorine-containing compound after about 70 hours in the exhaust gas decomposition system 100 may be about 35% or more. For example, the decomposition rate of the fluorine-containing compound after about 70 hours in the exhaust gas decomposition system 100 may be about 40% or more. For example, the decomposition rate of the fluorine-containing compound after about 70 hours in the exhaust gas decomposition system 100 may be about 45%. For example, the decomposition rate of the fluorine-containing compound after about 70 hours in the exhaust gas decomposition system 100 may be about 50% or more.

Referring to FIGS. 1 to 10, water-solubility of the fluorine-containing compound in the exhaust gas decomposition system 100 may be about 0.01 volume% or less at a temperature of 20°C and 1 atm. For example, the water-solubility of the fluorine-containing compound in the exhaust gas decomposition system 100 may be about 0.009 volume% at a temperature of 20°C and 1 atm. For example, the water-solubility of the fluorine-containing compound in the exhaust gas decomposition system 100 may be about 0.008 volume% at a temperature of 20°C at 1 atm. For example, the water-solubility of the fluorine-containing compound in the exhaust gas decomposition system 100 may be about 0.007 volume% at a temperature of 20°C at 1 atm. For example, the water-solubility of the fluorine-containing compound in the exhaust gas decomposition system 100 may be about 0.006 volume% at a temperature of 20°C at 1 atm. That is, the fluorine-containing compound may be substantially insoluble in liquid, such as water, containing a biological catalyst. Therefore, although the fluorine-containing compound is insoluble in liquid containing a biological catalyst, the exhaust gas decomposition system 100 provides improvements in terms of an area and a time of contact of the fluorine-containing compound with liquid containing a biological catalyst, thereby improving the decomposition rate of the fluorine-containing compound.

Referring to FIGS. 1 to 10, the liquid containing the biological catalyst in the exhaust gas decomposition system 100 may be a medium including at least one of an enzyme and a microorganism, wherein the enzyme cleaves F-C bonds. Here, the type of the medium is not particularly limited, and any medium capable of culturing a microorganism, such as an enzyme and a strain including the enzyme, may be used. For example, the medium may be a Luria-Bertani (LB) medium. Since the biological catalyst degrades F-C bonds, the fluorine-containing compound which contacts the biological catalyst may also be decomposed.

For example, the biological catalyst may include a microorganism belonging to the genus *Pseudomonas.* For example, a microorganism included in the biological catalyst may be a strain of *P. saitens,* for example strains KCTC 13107BP.

In addition, the biological catalyst may include a genetic modification that increases an activity level of 2-haloacid dehalogenase (HAD). The HAD may be of/classified-as EC 3.8.1.2. For example, the 2-HAD may be derived from strains selected from the group consisting of *Bacillus cereus, B. thuringiensis, B. megaterium,* and *Pseudomonas saitens,* but embodiments are not limited thereto. Any suitable strain available in the art may be used as the strain including the 2-HAD. For example, the recombinant microorganism may belong to the genus *Escherichia,* the genus *Bacillus,* or the genus *Pseudomonas,* but embodiments are not limited thereto. Any strain in the art suitable for use as the recombinant microorganism may be used.

Referring to FIGS. 1 to 10, in the exhaust gas decomposition system 100, oxygen may or may not be included in the reactor 10 depending on a type of the microorganism included in the biological catalyst. For example, when the microorganism included in the biological catalyst is anaerobic, the reactor 10 includes no oxygen or air. For example, when the microorganism included in the biological catalyst is aerobic, the reactor 10 includes oxygen or air therein, or may consist of oxygen or air.

Referring to FIGS. 1 to 10, in the exhaust gas decomposition system 100, the fluorine-containing compound may be a compound represented by one of Formulae 1 to 3:

<Formula 1>        $C(R_1)(R_2)(R_3)(R_4)$

<Formula 2>        $(R_5)(R_6)(R_7)C-[C(R_{11})(R_{12})]_n-C(R_8)(R_9)(R_{10})$

<Formula 3>        $S(R_{13})(R_{14})(R_{15})(R_{16})(R_{17})(R_{18})$.

In Formulae 1 to 3, n is an integer of 0 to 10,
$R_1$, $R_2$, $R_3$, and $R_4$ may each independently be fluorine (F), chlorine (Cl), bromine (Br), iodine (I), or hydrogen (H), wherein at least one selected from $R_1$, $R_2$, $R_3$, and $R_4$ is F,
$R_5$, $R_6$, $R_7$, $R_8$, $R_9$, $R_{10}$, $R_{11}$, and $R_{12}$ may each independently be F, Cl, Br, I, or H, wherein at least one selected from $R_5$, $R_6$, $R_7$, $R_8$, $R_9$, $R_{10}$, $R_{11}$, and $R_{12}$ is F, and
$R_{13}$, $R_{14}$, $R_{15}$, $R_{16}$, $R_{17}$, and $R_{18}$ may each independently be F, Cl, Br, I, or H, wherein at least one selected from $R_{13}$,

$R_{14}$, $R_{15}$, $R_{16}$, $R_{17}$, and $R_{18}$ is F.

Referring to FIGS. 1 to 10, in the exhaust gas decomposition system 100, the fluorine-containing compound may be a compound represented by one of Formulae 4 to 6:

<Formula 4>    $C(R_{21})(R_{22})(R_{23})(R_{24})$

<Formula 5>    $(R_{25})(R_{26})(R_{27})C\text{-}[C(R_{31})(R_{32})]_m\text{-}C(R_{28})(R_{29})(R_{30})$

<Formula 6>    $S(R_{33})(R_{34})(R_{35})(R_{36})(R_{37})(R_{38})$.

in Formulae 4 to 6,
m is an integer of 0 to 5,
$R_{21}$, $R_{22}$, $R_{23}$, and $R_{24}$ may each independently be F or H, wherein at least one selected from $R_{21}$, $R_{22}$, $R_{23}$, and $R_{24}$ is F,
$R_{25}$, $R_{26}$, $R_{27}$, $R_{28}$, $R_{29}$, $R_{30}$, $R_{31}$, and $R_{32}$ may each independently be F or H, wherein at least one selected from $R_{25}$, $R_{26}$, $R_{27}$, $R_{28}$, $R_{29}$, $R_{30}$, $R_{31}$, and $R_{32}$ is F, and
$R_{33}$, $R_{34}$, $R_{35}$, $R_{36}$, $R_{37}$, and $R_{38}$ may each independently be F or H, wherein at least one selected from $R_{33}$, $R_{34}$, $R_{35}$, $R_{36}$, $R_{37}$, and $R_{38}$ is F.

For example, in the exhaust gas decomposition system 100, the fluorine-containing compound may include at least one selected from $CH_3F$, $CH_2F_2$, $CHF_3$, $CF_4$, and $SF_6$.

According to another aspect of an embodiment, there is provided a complex an exhaust gas decomposition system also called an exhaust gas decomposition complex system including: the exhaust gas decomposition system of the invention, a first supplier or supply for supplying first fluid into the exhaust gas decomposition system, a second supplier or supply for supplying second fluid into the exhaust gas decomposition system; and a collector for collecting a decomposition product discharged from the exhaust gas decomposition system. The exhaust gas decomposition system may further include, in addition to the exhaust gas decomposition system, other devices, thereby further improving the decomposition rate of exhaust gas. The supplier is a system or unit that forwarding the first fluid into the exhaust gas decomposition system. The supplier may be a large tank containing the exhaust gas or vent line of an industrial plant. The collector is a system or unit that collects some or all of the decomposition product that is discharged from exhaust gas decomposition system. The collector may be a condenser or water bath.

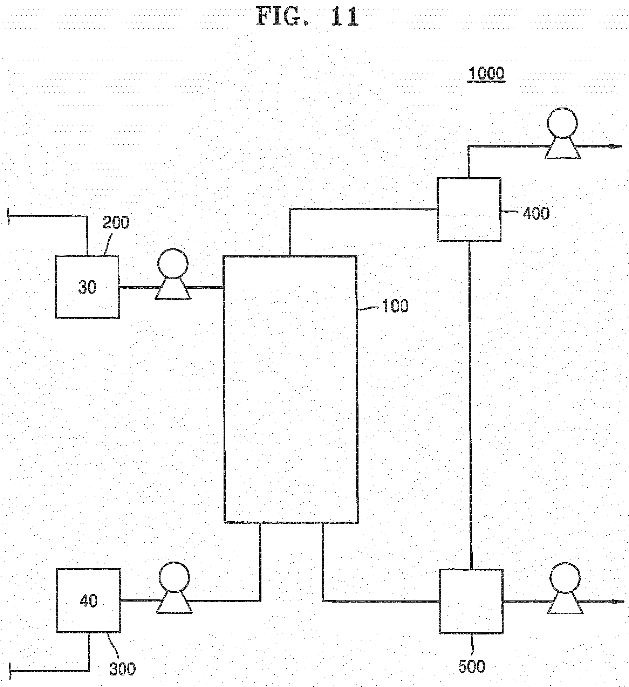

Referring to FIG. 11, an exhaust gas decomposition complex system 1000 includes the exhaust gas decomposition system 100, a first supplier 200 for supplying the first fluid 30 to the exhaust gas decomposition system 100, a second supplier 300 for supplying the second fluid 40 to the exhaust gas decomposition system 100, and first and second collectors 400 and 500 for collecting a decomposition product exhausted from the exhaust gas decomposition system 100.

Referring to FIG. 11, the first supplier 200 in the exhaust gas decomposition system 1000 may include a seed culture medium in which a biological catalyst, such as a microorganism, is cultured at a high concentration. When a microorganism is cultured at a high concentration, the decomposition rate of the fluorine-containing compound in the exhaust gas decomposition system 100 may be improved.

Referring to FIG. 11, the second supplier 300 in the exhaust gas decomposition system 1000 may include a pre-processor, such as an exhaust gas cleaning system commonly referred to as a "scrubber or fabric filter." The pre-processor means a system or unit that pretreat the exhaust gas entering the exhaust gas decomposition system by removing some large impurities from the exhaust gas. In the scrubber, impurities including solid particles, hydrochloric acid, hydrofluoric acid, and the like may be collected except that the fluorine-containing compound included in exhaust gas, to thereby purify the exhaust gas. As the impurities are collected in the scrubber, the purity of the fluorine-containing compound included in the second fluid 40 that is supplied to the exhaust gas decomposition system 100 increases, thereby improving the decomposition rate of the fluorine-containing compound.

Referring to FIG. 11, the first collector 400 in the exhaust gas decomposition system 1000 may include a condenser. Since the first collector 400 is a device for collecting gases exhausted from the exhaust gas decomposition system 100, a condenser or the like may be used to liquefy exhausted gases. For example, hydrofluoric acid (HF) gas has a boiling point as low as a temperature of 19.5°C. Thus, a condenser included in the first collector 400 may be used to liquefy HF gas exhausted from the exhaust gas decomposition system 100, by lowering the temperature thereof to 19°C or below, thereby collecting liquefied HF or supplying liquefied HF to the second collector 500.

Referring to FIG. 11, the second collector 500 in the exhaust gas decomposition system 1000 may include a residual liquid processor for neutralizing residual liquid discharged from at least one of the exhaust gas decomposition system 100 and the first collector 400. For example, a decomposition product discharged in a liquid state from a bottom portion of the exhaust gas decomposition system 100 may include liquid HF, and a decomposition product liquefied in the first collector 400 may also include liquid HF. In this regard, a base such as $Ca(OH)_2$ may be added to liquid HF to precipitate a salt of $CaF_2$ and thereby collect fluorine ions. Other than $CaF_2$, water ($H_2O$) which is harmless to the environment is produced.

Referring to FIG. 11, the decomposition product discharged from the exhaust gas decomposition system 100 of the exhaust gas decomposition complex system 1000 may include at least one selected from HF and hydrocarbon gases. A gaseous decomposition product may be exhausted through a top portion of the exhaust gas decomposition system 100 to be supplied to the first collector 400, whereas a decomposition product in liquid form may be exhausted through a bottom portion of the exhaust gas decomposition system 100 to be supplied to the second collector 500.

According to another aspect of an embodiment, there is provided strain KCTC 13107BP of *Pseudomonas saitens,* the strain being capable of reducing a concentration of fluorinated methane in a sample.

The strain may include a genetic modification that increases an activity level of the 2-HAD. The 2-HAD catalyzes a chemical reaction of 2-haloacid+$H_2O$ $\Leftrightarrow$ 2-hydroxy acid +halide. That is, two substrates of the 2-HAD are 2-haloacid and $H_2O$, and two products of the 2-HAD are 2-hydroxy acid and halide. The 2-HAD may belong to a family of hydrolases that act on a halide bond in a carbon-halide compound. However, reduction of the concentration of fluorinated methane or other fluorinated compound in a sample by the microorganism should not necessarily be interpreted as being limited to such a specific mechanism. The genetic modification may include increasing a number of copies of a gene encoding the 2-HAD. The gene encoding the 2-HAD may be an exogenous gene, and may be derived from the genus *Bacillus,* the genus *Pseudomonas,* the genus *Azotobacter*, the genus *Agrobacterium,* and the genus *Escherichia.* The gene encoding the 2-HAD may be derived from strains of *B. cereus, B. thuringiensis, B. megaferium*, or *Pseudomonas saitens* KCTC 13107BP. The 2-HAD may be an enzyme classified as EC 3.8.1.2.

The genetic modification may include increasing the number of copies of a gene encoding a polypeptide having a sequence identity of about 95% or higher with an amino acid sequence of SEQ ID NO: 1. The gene may have a sequence identity of about 95% or higher with a nucleotide sequence of SEQ ID NO: 2. The genetic modification may include introducing the gene encoding the 2-HAD, for example, via a vehicle such as a vector. The gene encoding the 2-HAD may exist within or outside the chromosome. A plurality of HAD genes or gene copies may be introduced, for example, 2 or more, 5 or more, 10 or more, 50 or more, 100 or more, or 1,000 or more.

The microorganism may reduce a concentration of fluorinated methane. The reduction may be performed by introducing a hydroxyl group into carbon by using a protein acting on C-F bonds or C-H bonds of fluorinated methane, or may be performed by accumulating fluorinated methane in cells of the microorganism. In addition, the reduction may include cleavage of C-F bonds of fluorinated methane, conversion of fluorinated methane into a different material, or accumulation of fluorinated methane in cells of the microorganism. The sample used herein may be liquid or gaseous. The sample may be factory waste water or waste gas. Any sample including fluorinated methane may be used in the art. Fluorinated methane may include $CF_4$, $CHF_3$, $CH_2F_2$, $CH_3F$, or a mixture thereof.

According to another aspect of an embodiment, there is provided a composition for reducing a concentration of fluorinated methane represented by $CH_nF_{4-n}$ (wherein n is an integer of 0 to 3), the composition including a strain KCTC 13107BP of *Pseudomonas saitens.*

Regarding the composition, the recombinant microorganism, the sample, and the fluorinated methane are the same defined in the description above.

Regarding the composition, the term "reducing" refers to reduction of a concentration of fluorinated methane in a sample, including complete removal of fluorinated methane in a sample. Here, the sample may be liquid or gaseous. The sample may or may not include the microorganism. The composition may further include a material that increases solubility of fluorinated methane in a medium or a culture product.

According to another aspect of an embodiment, there is provided a method of reducing a concentration of fluorinated methane in a sample, the method including: contacting a strain KCTC 13107BP of *Pseudomonas saitens* with a sample including fluorinated methane represented by $CH_nF_{4-n}$ (wherein n is an integer of 0 to 3).

Regarding the method, the microorganism and the sample including fluorinated methane represented by $CH_nF_{4-n}$ (wherein n is an integer of 0 to 3) are the same as defined in the description above.

Regarding the method, the contacting of the strain with the sample may be performed in a liquid phase environment or a solid phase environment. The contacting of the strain with the sample may be performed by, for example, contacting the sample with a culture product of a microorganism cultured on a medium. The culture may be performed under conditions in which a microorganism may grow. The contacting of the strain with the sample may be performed in a sealed container. The contacting of the strain with the sample may be performed when the growth stage of the microorganism is at an exponential phase or a stationary phase. The culture may be performed under aerobic or anaerobic conditions. The contacting of the strain with the sample may be performed under conditions in which a recombinant microorganism may survive in a sealed container. Such viable conditions may include a condition allowing proliferation of a recombinant microorganism or a condition allowing a recombinant microorganism to exist in a resting state. The contacting of the strain with the sample can be as described in reference to the various embodiments of system and system described above in connection with Figs. 1-14.

Regarding the method, the sample may be liquid or gaseous. The sample may be factory waste water or waste gas. The sample may not only passively contact the culture product of the microorganism, but may also actively contact the culture product of the microorganism. The sample may be, for example, subjected to a sparging process using a culture medium of the microorganism. That is, the sample may be blown through a medium or a culture medium. The sparging process may include blowing gas from the bottom of the medium or the culture medium to the top. The sparging process may include injecting of the sample while preparing droplets of the sample.

Regarding the method, the contacting of the strain with the sample may be performed in a batchwise or continuous manner. The contacting of the strain with the sample may include repeatedly or continuously contacting a sample with new, fresh microorganism. Thus, for instance, a sample having been contacted with the microorganism and having had the fluorine-containing compound in the sample reduced can be again contacted with a second, fresh microorganism and the fluorine-containing compound in the sample further reduced. The second microorganism can be of the same or different type as the first. Thus, for instance, the second microorganism can comprise a genetic modification that increases the activity level of the 2-HAD. Such contacting of the sample with the fresh microorganism may occur twice or more, for example, 2, 3, 5, or 10 times or more. The contacting of the sample with the fresh microorganism may be continuous or repeated for a period of time until a desired reduced concentration of fluorinated methane in the sample is achieved.

Regarding the method, the strain may further include a genetic modification that increases an activity level of the 2-HAD. Regarding the method, the genetic modification may include increasing the number of copies of a gene encoding the 2-HAD. Regarding the method, the gene encoding the 2-HAD may be an exogenous gene, and may be derived from the genus *Bacillus,* the genus *Pseudomonas,* the genus *Azotobacter,* the genus *Agrobacterium,* and the genus *Escherichia.* The gene encoding the 2-HAD may be derived from strains of *B. cereus, B. thuringiensis, B. megaterium,* or *Pseudomonas saitens* KCTC 13107BP. The 2-HAD may be classified as EC 3.8.1.2. A method of reducing a concentration of fluorinated methane in a fluid, the method comprises the system of above-mentioned, contacting a first fluid comprising a biological catalyst having 2-haloacid dehalogenase activity with a second fluid comprising fluorinated methane represented by $CH_nF_{4-n}$, wherein n is an integer of 0 to 3, to reduce the concentration of fluorinated methane in the second fluid. In the method, the biological catalyst may be a 2-haloacid dehalogenase enzyme or microorganism comprising a 2-haloacid dehalogenase enzyme. In the method, the microorganism may be Pseudomonas. In the method, the microorganism may be Pseudomonas saitens. In the method, the strain of Pseudomonas may be a strain of KCTC 13107BP strain of Pseudomonas saitens.

Hereinafter, the present inventive concept will be described in more detail with reference to Examples. However, these Examples are provided for illustrative purposes only, and the invention is not intended to be limited by these Examples.

Preparation Example 1: Selection of *Pseudomonas saitens* strain capable of decomposing tetrafluoromethane (CR$_4$)

In the present Example, microorganisms capable of reducing a concentration of $CF_4$ in semiconductor factory waste water were selected.

Sludge in the waste water discharged from a Samsung Electronics factory (at Giheung, Korea) was applied to an agar plate containing a medium containing no carbon (supplemented with 0.7 g/L of $K_2HPO_4$, 0.7 g/L of $MgSO_4 \cdot 7H_2O$, 0.5 g/L of $(NH_4)_2SO_4$, 0.5 g/L of $NaNO_3$, 0.005 g/L of NaCl, 0.002 g/L of $FeSO_4 \cdot 7H_2O$, 0.002 g/L of $ZnSO_4 \cdot 7H_2O$, 0.001 g/L of $MnSO_4$, and 15 g/L of agar). The agar plate was placed in a GasPak™ Jar (BD Medical Technology), and the jar was filled with 99.9 v/v% $CF_4$ and sealed. The cells were then cultured at a temperature of 30°C under anaerobic conditions. Single colonies formed after culture were cultured using a high throughput screening (HTS) system (Thermo Scientific/Liconic/Perkin Elmer), and then, each of the single colonies was inoculated on a 97-well microplate containing 100 μL/well of an LB medium. The colonies were then subjected to stationary culture at a temperature of 30°C for 72 hours under aerobic conditions. Here, the absorbance of the colonies was measured at 600 nm every 12 hours so that the growth ability of the colonies could be observed. The LB medium used herein contains 10 g/L of tryptone, 5 g/L of yeast extract, and 10 g/L of NaCl.

The top 2% of strains showing excellent growth ability were selected, and each strain was inoculated into a 75 mL glass serum bottle containing 10 mL of an LB medium to have OD600 of 0.5. Then, the glass serum bottle was sealed, and $CF_4$ was injected thereinto with a syringe so that 1,000 ppm of $CF_4$ gas was in the glass serum bottle. The glass serum bottle was incubated in a shaking incubator at a temperature of 30°C for 4 days while being stirred at a speed of 230 rpm, and then the amount of $CF_4$ in the headspace thereof was analyzed.

For analysis, 0.5 ml of $CF_4$ was collected from the headspace using a syringe, and injected into a gas chromatography (GC) column (Agilent 7890, Palo Alto, CA, USA). The injected $CF_4$ was separated through a CP-PoraBOND Q column (25 m length, 0.32 mm i.d., 5 um film thickness, Agilent), and changes in the $CF_4$ concentration were analyzed by MSD (Agilent 5973, Palo Alto, CA, USA). As a carrier gas, helium was used, and applied to the column at a flow rate of 1.5 ml/min. GC conditions were as follows: an inlet temperature was 250°C, and an initial temperature was maintained at 40°C for 2 minutes and then raised to 290°C at a rate of 20°C/min. MS conditions were as follows: ionization energy was 70 eV, an interface temperature was 280°C, an ion source temperature was 230°C, and a quadrupole temperature was 150°C. Unless otherwise mentioned, analysis of gas such as $CHF_3$, $CHCl_3$, and $CF_4$ was performed by using the above method. As a control group, 1000 ppm of $CF_4$ was incubated without cells under the same conditions as described above, and then measured.

As a result, it was confirmed that the $CF_4$ concentration was reduced by 10.4% in the selected microorganism relative to the control group having no cells. The selected microorganism had decomposition activity of 0.005 umol/g-cell/min. To identify the selected strain, a 16s rRNA gene (SEQ ID NO: 3) was amplified using the genome of the separated cell as a template. Here, the nucleotide sequences of the 16s rRNA gene were analyzed by BLAST Assembled Genomes.

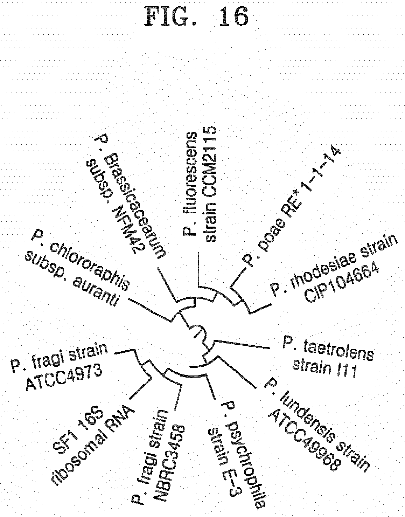

A final size of assembled genomes was 5.1 Mb, and a GC content thereof was 59.14%. As a result of automated annotation using the Prokaryotic Genome Annotation Pipeline, a total of 328 genes, 25 rRNA operons, 73 tRNAs, and 1 tmRNA were found to be present. As a result of analyzing a phylogenetic tree, it was confirmed that the separated microorganism belongs to genus *Pseudomonas.* FIG. 16 is a diagram showing the phylogenetic tree of the separated microorganism. However, the selected microorganism included no sequence having an exact sequence match with that of any previously known species belonging to the genus *Pseudomonas.*

The selected microorganism was newly designated as a strain of *Pseudomonas saitens* (hereinafter, referred to as "SF1"), and was deposited with and accepted by the Korea Collection for Type Culture (KCTC) on September 12, 2016, under the Access number of KCTC 13107BP.

Preparation Example 2: Preparation of microorganism into which gene facilitating decomposition of $CF_4$ is introduced

1. Amplification of an HAD gene derived from *Bacillus cereus* (hereinafter, referred to as BC HAD), and introduction of the gene to *Escherichia coli* (*E. coli*).

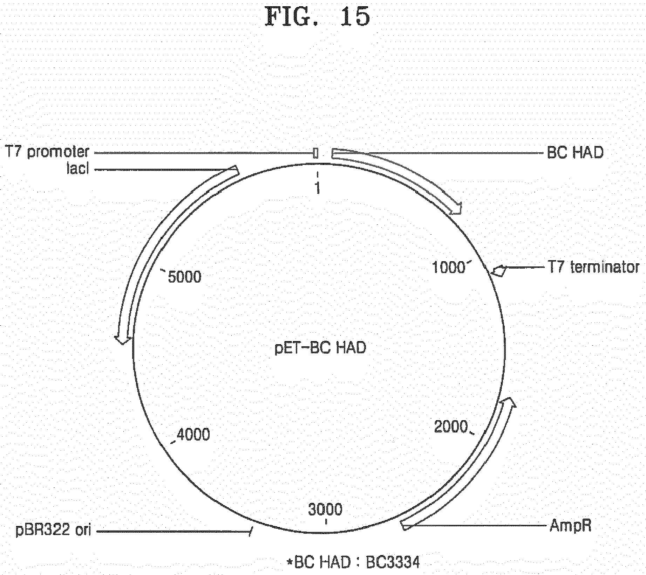

*B. cereus* (KCTC 3624) was cultured overnight in an LB medium at a temperature of 30°C while being stirred at 230 rpm, and then, the genomic DNA was isolated therefrom using a total DNA extraction kit (Invitrogen Biotechnology). Then, PCR was performed using the isolated genomic DNA as a template and a set of primers having nucleotide sequences listed in Table 1, to amplify and obtain a *BC3334* gene. A pET-BC HAD vector was prepared by using an InFusion Cloning Kit (Clontech Laboratories, Inc.), wherein the BC HAD gene which was amplified by PCR was ligated with pETDuet-1 (Novagen, Cat. No. 71146-3) which had been digested with restriction enzymes Ncol and HindIII. FIG. 15 shows a vector map of the pET-BC HAD vector. The BC3334 gene had an amino acid sequence of SEQ ID NO: 1 and a nucleotide sequence of SEQ ID NO: 2.

Next, pET-BC3334, which is the pET-BC HAD vector, was introduced into an *E. coli* BL21 strain by a heat shock method, and then the microorganism was cultured on an LB plate containing ampicillin (100 μg/mL). A strain showing ampicillin resistance was selected. The finally selected strain was then designated as a recombinant *E. coli* BL21/pET-BC3334.

[Table 1]

| BC HAD gene | Primer sequence (SEQ ID NO.) |
|---|---|
| BC3334 | Forward: SEQ ID NO: 4<br>Reverse: SEQ ID NO: 5 |

Example 1: Straight glass tube cooler tilted at an angle of 40° and SF1 strain

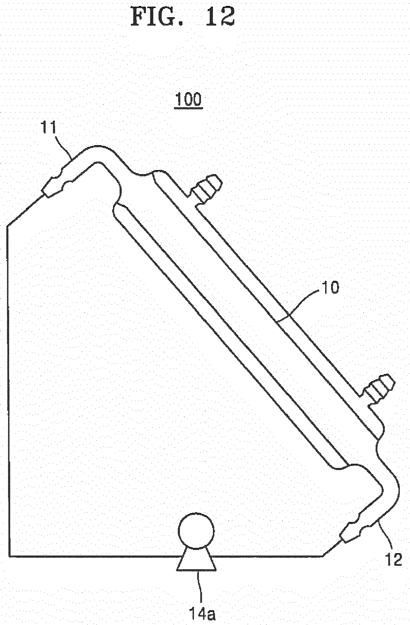

As shown in FIG. 12, 60 ml of an LB medium and 1,000 ppm of $CF_4$ gas were injected into a straight glass tube cooler (length of reactor: 500 mm, volume of inner tube: 300 mL, diameter of inner tube: 35 mm, and diameter of outer tube: 60 mm) that was sterilized at a high temperature and tilted by at an angle of 40° with respect to a vertical direction, and then, the LB medium was circulated. The LB medium was supplied into an inlet at an upper portion of the straight glass tube cooler, flowed along an inner wall of the straight glass tube cooler, and then, discharged through an outlet at a bottom portion of the straight glass tube cooler. The discharged LB medium was re-supplied back into the inlet along a circulation line. Although not shown in FIG. 12, an outer jacket of the straight glass tube cooler was connected to a constant temperature zone for temperature maintenance. The circulation rate of the LB medium was 4 mL/min, and the temperature inside the straight glass tube cooler was maintained at 30°C. After 48 hours, the amount of $CF_4$ gas in the straight glass tube cooler was confirmed by gas chromatography mass-spectrometry (GC-MS). No change in the amount of $CF_4$ gas was observed.

Next, the strain of *Pseudonmonas saitens* selected according to Preparation Example 1 was inoculated with a syringe into the LB medium in the straight glass tube cooler. The initial concentration of the inoculated strain in the LB medium was 0.5 at OD of 600 nm. Then, the LB medium to which the strain was inoculated was circulated. The circulation rate of the LB culture medium was 4 mL/min, and the temperature inside the straight glass tube cooler was maintained at 30°C. After 66 hours, the amount of $CF_4$ gas in the straight glass tube cooler was confirmed by GC-MS. Here, the

decomposition rate of $CF_4$ was calculated according to Equation 1, and results thereof are shown in Table 2.

$$\text{<Equation 1>}$$

$$\text{Decomposition rate of } CF_4 = [(\text{initial amount of } CF_4 - \text{amount of } CF_4 \text{ after 66 hours}) / \text{initial amount of } CF_4] \times 100$$

Example 2: Vertical glass Dimroth screwed reflux condenser and SF1 strain

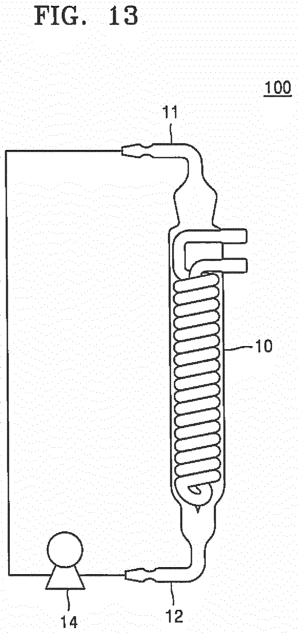

In the same manner as in Example 1, except that a vertical glass Dimroth screwed reflux condenser (length of reactor: 350 mm, diameter of outer tube: 35 mm, and volume of inner tube: 200 mL) shown in FIG. 13 sterilized at a high temperature was used instead of the straight glass tube cooler, and 40 ml of the LB medium and 1,000 ppm of $CF_4$ gas were injected into the vertical glass Dimroth screwed reflux condenser.

After 66 hours, the amount of $CF_4$ gas in the vertical glass Dimroth screwed reflux condenser was confirmed by GC-MS. Here, the decomposition rate of $CF_4$ was calculated according to Equation 1, and results thereof are shown in Table 2.

Example 3: Vertical-straight filled glass tube cooler and SF1 strain

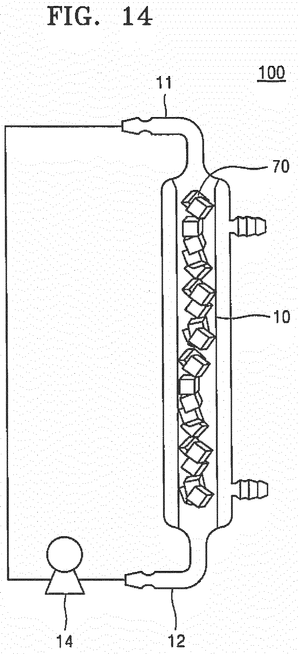

In the same manner as in Example 1, except that, as shown in FIG. 14, 30 porous filling materials (10 mm x 10 mm x 10 mm) formed of polypropylene were added to the straight glass tube cooler, and 60 ml of the LB medium and 1,000 ppm of $CF_4$ gas were injected into the straight glass tube cooler filled with fillers.

After 66 hours, the amount of $CF_4$ gas in the straight glass tube cooler filled with fillers was confirmed by GC-MS. Here, the decomposition rate of $CF_4$ was calculated according to Equation 1, and results thereof are shown in Table 2.

Comparative Example 1: Glass serum bottle and SF1 strain

10 mL of the LB medium to which the strain of Example 1 was inoculated and 1,000 ppm of $CF_4$ gas were added to a 75 ml glass serum bottle. After maintaining the glass serum bottle for 96 hours in a shaking incubator at a speed of 230 rpm and at a temperature of 30°C, the amount of $CF_4$ gas in the glass serum bottle was confirmed by GC-MS. Here, the decomposition rate of $CF_4$ was calculated according to Equation 1, and results thereof are shown in Table 2. The initial concentration of the inoculated strain in the LB medium was 0.5 at OD of 600 nm.

[Table 2]

|  | Residence time [hr] | Decomposition rate of $CF_4$ [%] |
|---|---|---|
| Example 1 | 66 | 21.8 |
| Example 2 | 66 | 34.7 |
| Example 3 | 66 | 36.8 |
| Comparative Example 1 | 96 | 10.5 |

As shown in Table 2, it was confirmed that the decomposition rate of $CF_4$ was significantly improved in the fluorinated gas decomposition devices of Examples 1 to 3 in which the strain-inoculated LB medium was circulated, relative to the fluorinated gas decomposition device of Comparative Example 1 in which the strain-inoculated LB medium was simply stirred. Such improved decomposition rates of $CF_4$ may result from an increased time and/or area of contact of the $CF_4$ gas with the strain-inoculated LB medium, as the strain-inoculated LB medium existed in the form of a thin film on the inner wall of the cooler, on the surface of the screw tube, or on the surface of the porous filler.

Example 4: Straight glass tube cooler tilted at an angle of 40° and *E. coli* including *BC3334* introduced thereto

As shown in FIG. 12, 60 ml of an LB medium and 1,000 ppm of $CF_4$ gas were injected into a straight glass tube cooler (length of reactor: 500 mm, volume of inner tube: 300 mL, diameter of inner tube: 35 mm, and diameter of outer tube: 60 mm) that was sterilized at a high temperature and tilted by at an angle of 40° with respect to a vertical direction (50° from the plane of the Earth's surface), and then, the LB medium was circulated. The LB medium was supplied into an inlet at an upper portion of the straight glass tube cooler, flowed along an inner wall of the straight glass tube cooler, and then, discharged through an outlet at a bottom portion of the straight glass tube cooler. The discharged LB medium was re-supplied back into the inlet along a circulation line. Although not shown in FIG. 12, an outer jacket of the straight glass tube cooler was connected to a constant temperature zone for temperature maintenance. The circulation rate of the LB medium was 4 mL/min, and the temperature inside the straight glass tube cooler was maintained at 30°C. After 48 hours, the amount of $CF_4$ gas in the straight glass tube cooler was confirmed by GC-MS. As a result, no change in the amount of $CF_4$ gas was observed.

Next, the strain of *E. coli* in which 2-HAD BC3334 gene was introduced in Preparation Example 2 was inoculated with a syringe into the LB medium in the straight glass tube cooler. The initial concentration of the inoculated strain in the LB medium was 0.5 OD at 600 nm. Then, the LB medium to which the strain was inoculated was circulated. The circulation rate of the LB culture medium was 4 mL/min, and the temperature inside the straight glass tube cooler was maintained at 30°C. After 66 hours, the amount of $CF_4$ gas in the straight glass tube cooler was confirmed by GC-MS. Here, the decomposition rate of $CF_4$ was calculated according to Equation 1, and results thereof are shown in Table 3.

Example 5: Vertical glass Dimroth screwed reflux condenser and *E. coli* to including BC3334 introduced thereto

In the same manner as in Example 1, except that a vertical glass Dimroth screwed reflux condenser (length of reactor: 350 mm, diameter of outer tube: 35 mm, and volume of inner tube: 200 mL) shown in FIG. 13 sterilized at a high temperature was used instead of the straight glass tube cooler, and 40 ml of the LB medium and 1,000 ppm of $CF_4$ gas were injected into the vertical glass Dimroth screwed reflux condenser.

After 66 hours, the amount of $CF_4$ gas in the vertical glass Dimroth screwed reflux condenser was confirmed by GC-MS. Here, the decomposition rate of $CF_4$ was calculated according to Equation 1, and results thereof are shown in Table 3.

Example 6: Vertical-straight filled glass tube cooler and *E. coli* to including *BC3334* introduced thereto

In the same manner as in Example 1, except that, as shown in FIG. 14, 30 porous filling materials (10 mm x 10 mm x 10 mm) formed of polypropylene substances were added to the straight glass tube cooler, and 60 ml of the LB medium and 1,000 ppm of $CF_4$ gas were injected into the straight glass tube cooler filled with fillers.

After 66 hours, the amount of $CF_4$ gas in the straight glass tube cooler filled with fillers was confirmed by GC-MS. Here, the decomposition rate of $CF_4$ was calculated according to Equation 1, and results thereof are shown in Table 3.

Comparative Example 2: Glass serum bottle and *E. coli* to including BC3334 introduced thereto

10 mL of the LB medium to which the strain of Example 1 was inoculated and 1,000 ppm of $CF_4$ gas were added to a 75 ml glass serum bottle. After maintaining the glass serum bottle for 96 hours in a shaking incubator at a speed of 230 rpm and at a temperature of 30°C, the amount of $CF_4$ gas in the glass serum bottle was confirmed by GC-MS. Here, the decomposition rate of $CF_4$ was calculated according to Equation 1, and results thereof are shown in Table 1. The initial concentration of the inoculated strain in the LB medium was 0.5 at OD of 600 nm.

[Table 3]

|  | Residence time [hr] | Decomposition rate of $CF_4$ [%] |
|---|---|---|
| Example 4 | 66 | 18.9 |
| Example 5 | 66 | 30.4 |
| Example 6 | 66 | 29.8 |
| Comparative Example 2 | 96 | 5.67 |

As shown in Table 3, it was confirmed that the decomposition rate of $CF_4$ was significantly improved in a shorter time in the fluorinated gas decomposition devices of Examples 4 to 6 in which the strain-inoculated LB medium was circulated, relative to the fluorinated gas decomposition device of Comparative Example 2 in which the strain-inoculated LB medium was simply stirred. Such improved decomposition rates of $CF_4$ may result from an increased time and/or area of contact of the $CF_4$ gas with the strain-inoculated LB medium, as the strain-inoculated LB medium existed in the form of a medium thin film on the inner wall of the cooler, on the surface of the screw tube, or on the surface of the porous filler.

As described above, the circulation of at least one of a biological catalyst and a fluorine-containing compound in an exhaust gas decomposition system may lead to improvement of a decomposition rate of the fluorine-containing compound.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Any combination of the above-described elements in all possible variations thereof is encompassed by the application unless otherwise indicated herein or otherwise clearly contradicted by context.

**Claims**

1. An exhaust gas decomposition system comprising:

   one or more bioreactors, each comprising one or more first inlets, and one or more first outlets;
   a supply of a first fluid connected to at least one of the one or more first inlets of each of the one or more bioreactors;
   a second fluid within the one or more bioreactors;
   wherein one of the first fluid and the second fluid comprises a biological catalyst that decomposes a fluorine-containing compound, and the other comprises the fluorine-containing compound and wherein the fluorine-containing compound is decomposed by contact of the first and second fluid;
   wherein, the first fluid supplied to the one or more first inlets, flows in a first direction in each of the one or more bioreactors, contacts the second fluid, and is exhausted through the one or more first outlets.

2. The exhaust gas decomposition system of claim 1, wherein the first fluid is a liquid containing the biological catalyst, and the second fluid is a gas comprising the fluorine-containing compound.

3. The exhaust gas decomposition system of claim 1 or 2, wherein at least one of the one or more bioreactors further comprises a first circulation line for re-supplying at least a portion of the first fluid discharged through at least one of the one or more first outlets back to at least one of the one or more first inlets.

4. The exhaust gas decomposition system of any one of claims 1 to 3, wherein the system further comprises:

   one or more second inlets, and
   one or more second outlets;
   a supply of the second fluid connected to at least one of the one or more second inlet of each of the one or more bioreactors;
   wherein the second fluid supplied to the second inlet is exhausted through the second outlet and flows in a direction opposite the first direction.

5. The exhaust gas decomposition system of anyone of claims 1 to 4, wherein the bioreactor comprises a bed on which a thin film of the first fluid is formed.

6. The exhaust gas decomposition system of any one of claims 1 to 5, wherein the interior of one or more of the reactors comprises a structure that increases an area of contact between the first fluid with the second fluid in comparison with a structure-free bioreactor.

7. The exhaust gas decomposition system of claim 6, wherein the structure comprises at least one of a filler and a reflux tube, and/or wherein the structure is porous.

8. The exhaust gas decomposition system of any one of claims 1 to 7, wherein the exhaust gas decomposition apparatus further comprises one or more sprayers for spraying the first fluid into a fluid reaction zone, said one or more sprayers being connected to the one or more first inlets.

9. The exhaust gas decomposition system of any one of claims 1 to 8, wherein
   at least one of the one or more first inlets opens into an upper portion of an inner space of the one or more bioreactors;
   a fluid collection zone for collecting the first fluid is disposed at a lower portion of the inner space of each of the one or more bioreactors,
   and at least one of the one or more second inlets opens into the lower portion of the bioreactor.

10. The exhaust gas decomposition system of any one of claims 5 to 9, wherein at least one of the one or more bioreactor is disposed at an angle of about 30° to about 150° relative to the surface of the earth.

11. The exhaust gas decomposition system of any one of claims 1 to 10, wherein the system comprises two or more bioreactors that are connected to one another in series or in parallel.

12. The exhaust gas decomposition system of any one of claims 1 to 11, further comprising:

   a first supplier for supplying the first fluid into the exhaust gas decomposition system;

a second supplier for supplying the second fluid into the exhaust gas decomposition system; and
first and second collectors for collecting a decomposition product discharged from the exhaust gas decomposition system,
wherein the first supplier comprises a culture medium, the second supplier comprises a pre-processor, and the first collector comprises a condenser.

13. A strain of *Pseudomonas saitens* deposited under KCTC 13107BP.

14. The strain of claim 13, wherein the strain further comprises a genetic modification that increases an activity of 2-haloacid dehalogenase (HAD).

15. A method of reducing a concentration of fluorinated methane in a sample, the method comprising:

contacting the strains of claims 13 or 14 with a sample comprising fluorinated methane represented by $CH_nF_{4-n}$, wherein n is an integer of 0 to 3, to reduce a concentration of fluorinated methane in a sample.

wherein HAD is preferably classified as EC 3.8.1.2.

# FIG. 1

FIG. 2

FIG. 3

FIG. 4

# FIG. 5

# FIG. 6

# FIG. 7

100

D

H

10

27

## FIG. 8

FIG. 9

# FIG. 10

# FIG. 11

FIG. 12

FIG. 13

FIG. 14

# FIG. 15

T7 promoter
lacl

BC HAD

1

5000

1000

T7 terminator

pET-BC HAD

2000

4000

3000

pBR322 ori

AmpR

*BC HAD : BC3334

# FIG. 16